# EUROPEAN PATENT SPECIFICATION

(11) **EP 1 933 853 B1**
(45) Date of publication and mention of the grant of the patent: **17.07.2013**
(21) Application number: 06774917.6
(22) Date of filing: 01.09.2006
(51) Int. Cl.: A61K 35/16, A61P 19/00, A61P 17/02, A61P 29/00, A61K 33/24

(54) **TISSUE DISRUPTION TREATMENT AND COMPOSITION FOR USE THEREOF**
BEHANDLUNG VON GEWEBEDISRUPTION UND ZUSAMMENSETZUNG ZUR ANWENDUNG DAFÜR
TRAITEMENT DE L'INTERRUPTION DE TISSU ET COMPOSITION À EMPLOYER DANS CE TRAITEMENT

(30) Priority: 01.09.2005 US 218382
(43) Date of publication of application: 25.06.2008
(73) Proprietor: Cambridge Scientific Pty Ltd., Leederville, WA 6007 (AU)
(72) Inventor: EDWARDS, Jeffrey D, Claremont, Western Australia 6010 (AU); EIJKENBOOM, Maud Louisa Johanna Maria, Melville, Western Australia 6156 (AU)
(74) Representative: Ziebig, Marlene
(86) International application number: PCT/AU2006/001288
(87) International publication number: WO 2007/025351

(56) References cited:
- EP-A1- 0 061 556
- EP-A1- 0 417 002
- EP-A2- 0 956 869
- WO-A1-2006/084334
- WO-A1-2006/084334
- RU-C1- 2 221 456
- US-A- 2 579 367
- US-A- 3 152 955
- US-A- 3 672 954
- US-A- 5 888 522

## Description

### FIELD

The present invention relates to an agent having activity in the treatment of a tissue disruption. In particular the present invention relates to a composition comprising an effective amount of an active fraction having tissue healing properties, wherein said active fraction is separated from a mixture of plasma and/or serum and at least one metal, metal ion or metal salt thereof and wherein said mixture has been denatured.

### BACKGROUND

The treatment of tissue disruptions such as sunburn, soft and connective tissue injury and wounds can be impeded by the lack of effective therapeutics. Part of the problem is a lack of understanding of the process of healing.

Wound healing is usually a coordinated, stereotyped sequence of events that includes (a) tissue disruption and loss of normal tissue architecture, (b) cell necrosis and haemorrhage; hemostasis (clot formation), (c) infiltration of segmented and mononuclear inflammatory cells, with vascular congestion and tissue oedema, (d) dissolution of the clot as well as damaged cells and tissues by mononuclear cells (macrophages), and (e) formation of granulation tissue (fibroplasia and angiogenesis). This sequence of cellular events has been observed in wounds from all tissues and organs generated in a large number of mammalian species (Gailet et al., 1994, Curr. Opin. Cell. Biol. 6:717-725). Therefore, the cellular sequence described above is a universal aspect of the repair of all mammalian tissues.

Moreover, the process of wound healing involves cytokines, enzymes and growth factors, such as TNF-α, TNF-α-converting enzyme (TACE), and caspases. For example, TNF-α is released in the skin after UV light exposure and, following binding of membrane receptors, initiates signal transduction. Similarly, the expression of the TNF-α receptor (TNF-αR) has been reported to be modified by ultraviolet light. The expression of TNF-α is also induced rapidly following brain trauma. TACE inhibitors reduce the secretion of TNF-alpha, so that TNF-alpha remains bound to the cell membrane. Caspases have been strongly linked to apoptosis, such as in animal models for traumatic brain injury. Cytokine inhibitors and inhibitors of apoptosis can counteract the secondary injury that often occurs after an initial tissue disruption, and thus decrease the extent of tissue disruption that would have otherwise occurred with this treatment.

Many of the current treatment compositions for tissue disruptions have difficulties addressing the optimum requirements. For example, with respect to the treatment of wounds (one type of tissue disruption) the optimum requirements are acceleration of the rate of wound contraction, increasing the rate of epithelialisation and increasing the rate of maturation of granulation material, thereby ultimately reducing the time to full maturity of the healed wound.

Similar problems have also been experienced with other types of tissue disruptions. For example, burns have been unsuccessfully treated. With respect to deep soft tissue injuries, previous treatments have included injections of various materials to repair or swell soft tissues. Some of the agents used include liquid silicone, collagen in various forms such as chemically cross-linked and fibrous forms, and hyaluronic acid.

Unfortunately, none of these procedures or materials are considered to be ideal owing to short-comings in effectiveness or efficacy. For example, liquid silicone was banned by the FDA when it was discovered that it could migrate to distant parts of the body and cause physiological and clinical problems.

In W02006/084334 it is disclosed a composition that is suitable for relief of pain, fever and inflammation in a variety of conditions including burns.

EP0956869 discloses the use of proteins extracted from plasma in combination with metals for the treatment of skin injuries. The proteins have have been precipitated from plasma, which had been frozen at -80°C.

US2579367 discloses a wound dressing for treatment of wounds. The wound dressing is made of a proteinaceous paste made by mixing the protein with sodium lauryl sulfate, sodium lactate, NaOH and water, and subsequently autoclaving the mixture. During the production of the protein solution hydrolysis occurs. The protein source can be blood serum. A second sterilisation (heating to 121°C) can take place. A second separate layer is present in the wound dressing, said layer comprising a coagulant which can be a metal.

EP0417002 uses whole blood for the preparation of wound dressings which is treated with papain or pepsin

US3672954 discloses a hemolysed blood extract with healing activity on wounds, which is obtained after hemolysis, hydrolysis (papain) and then heated to 80°C

US5888522 discloses a soybean hydrolysate (enzymatic digest) and its use in treatment of wounds. The enzymatic digest is mixed with a salt to precipitate the peptides.

It is therefore desirable to have a treatment composition that can be used to treat tissue disruptions including soft and connective tissue injuries, deep tissue injuries, surface wounds and open wounds, wherein the time to full maturity of the injury is reduced by halting primary as well as secondary damage, and accelerating the rate of tissue repair.

Following extensive biochemical laboratory research, the present inventors have developed a composition capable of overcoming or at least alleviating some of the problems associated with prior art tissue disruption treatments.

### SUMMARY

The present invention provides a composition for use in treatment of wounds in a subject by topical administration as defined in the claims. Said composition comprises at least a fraction of a wound healing mixture, wherein said wound healing mixture is produced by:
- mixing plasma and/or serum with at least one metal, metal ion or metal salt thereto and heating the resulting mixture to at least 50°C;
- adding one or more proteases;
- heating the resultant mixture to a temperature of 80°C to 150°C; and
- allowing the mixture to cool to produce the wound healing mixture.

The plasma or serum may be obtained from any animal source. Preferably, the plasma or serum is isolated from an animal selected from the group consisting of human, equine, bovine, ovine, murine, caprine and canine.

In some embodiments, the plasma and/or serum is dried and lyophilised before use.

Once the plasma and/or serum has been obtained it is mixed with at least one metal, metal ion or metal salt thereof. The metal, metal ion or metal salt thereof can be any metal. In some embodiments, the metal is selected from the group consisting of nickel, sodium, copper, zinc, cobalt, iron, magnesium, manganese, potassium, silver and mercury, ions or salts thereof and mixtures thereof.

Once the metal, metal ion or metal salt thereof has been mixed with the plasma and/or serum, it is heated to at least 50°C. Preferably, the mixture is heated to about 65°C.

One or more proteases, such as trypsin, is/are added after heating. The resultant mixture will again be heated then allowed to cool to produce a mixture that is capable of healing tissue disruptions such as wounds.

The second heating step is carried out between about 80°C and about 150°C, more preferably between about 90°C and about 130°C and most preferably, about 120°C.

The wound healing mixture of the present invention can be used directly or further separated to produce a more defined fraction having healing properties.

The composition of the present invention comprises at least a fraction of the wound healing mixture as described above. In some embodiments the composition of the present invention is optionally admixed with a pharmaceutical carrier. Any pharmaceutical carrier known in the art may be used.

In a further aspect the present invention provides a wound dressing comprising a composition of the invention for use in treatment of wounds.

It is further contemplated that the active fraction of plasma and/or serum and at least one metal, metal ion or salt thereof can also be used to coat medical devices used in the treatment of diseases or disorders. The medical devices that can be thus coated are, for example, catheters, guide channels, probes, cardiac valves, soft tissue replacements, replacements of animal origin, artificial tendons, bone and cardiovascular replacements, contact lenses, blood oxygenators, artificial kidneys, hearts, pancreas and livers, blood bags, syringes, surgical instruments, filtration systems, laboratory instruments, containers for cell and tissue culture and regeneration, supports for peptides, proteins and antibodies.

It is further contemplated that a therapeutic composition and/or wound dressing of the present invention may further comprise compounds including but not limited to antimicrobials, anti-virals, growth factors, anti-dehydration compounds, coagulant agents such as Factor Xa, antiseptics, or other compounds suitable for biomedical and/or veterinary uses.

### BRIEF DESCRIPTION OF THE FIGURES

Figure 1 shows regions of human skin exposed to UV light treated with a composition of the present invention as compared to untreated skin.
Figure 2 shows the same skin as in Figure 1, but 7 weeks post-exposure.

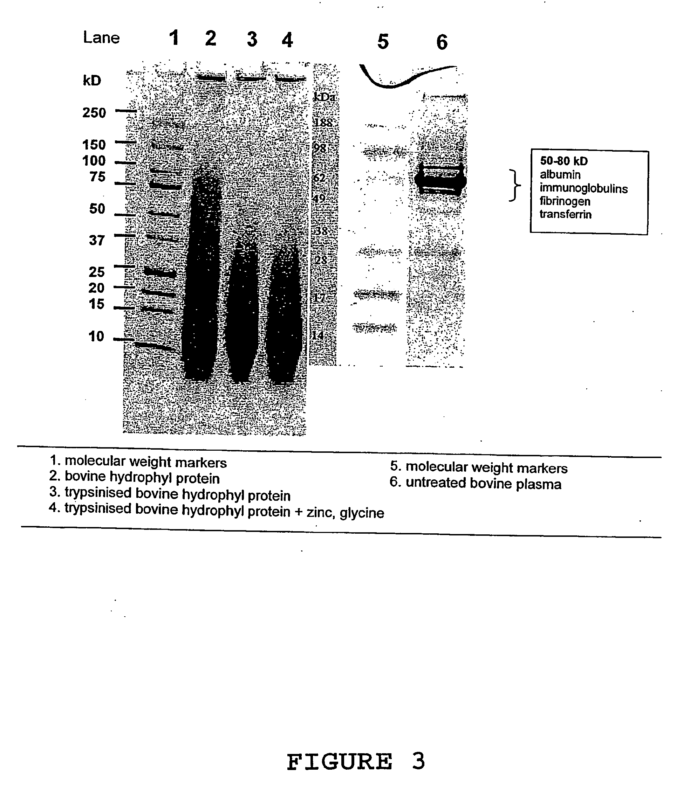

Figure 3 (left panel) shows a 10-20% SDS-PAGE Tricine gradient gel. Proteins were stained with Coomassie blue. Lane 1 contains molecular weight markers. Lanes 2 shows bovine soluble protein prior to trypsinisation and lane 3 after trypsinisation, as indicated. This gel shows that the majority of proteins in the preparation are in a size range less than 50 kilodaltons. Figure 3 (right panel) shows a 12% SDS-PAGE Tricine gel. Proteins were silver-stained Lane 4 contains molecular weight markers. Lane 5 shows untreated bovine plasma. This gel shows that the majority of proteins in unpurified bovine plasma are in a size range of 50-80 kilodaltons.
Figure 4 shows bovine plasma by 2-D electrophoresis map using the method of Talamo et al., 2003, Proteomics, 3:440-460.
Figure 5 shows the effect of one form of the composition of the present invention comprising zinc chloride, glycine and trypsinised protein on the TNF-α production by LPS-stimulated human monocytes.
Figure 6 shows the effect of the composition of the present invention, containing copper as the metal-containing solution, on the TNF-α production by LPS-stimulated human monocytes.
Figure 7 shows the effect of reduced concentrations of one form of the composition of the present invention comprising zinc chloride, glycine and trypsinised protein on the TNF-α production by LPS-stimulated human monocytes.
Figure 8 shows the titration of the effect of different concentrations of the composition of the present invention. The purpose was to demonstrate that the test sample comprising zinc chloride, glycine and trypsinised protein does not compete with the FCS which is being used in the culture medium.
Figure 9 shows the effect of the composition on the metabolism of cells *in vitro,* with or without LPS challenge, on a non-radioactive proliferation assay (CellTiter 96® AQᵤₑₒᵤₛ Assay). The purpose was to demonstrate that the test composition does not reduce the metabolism of the cells.

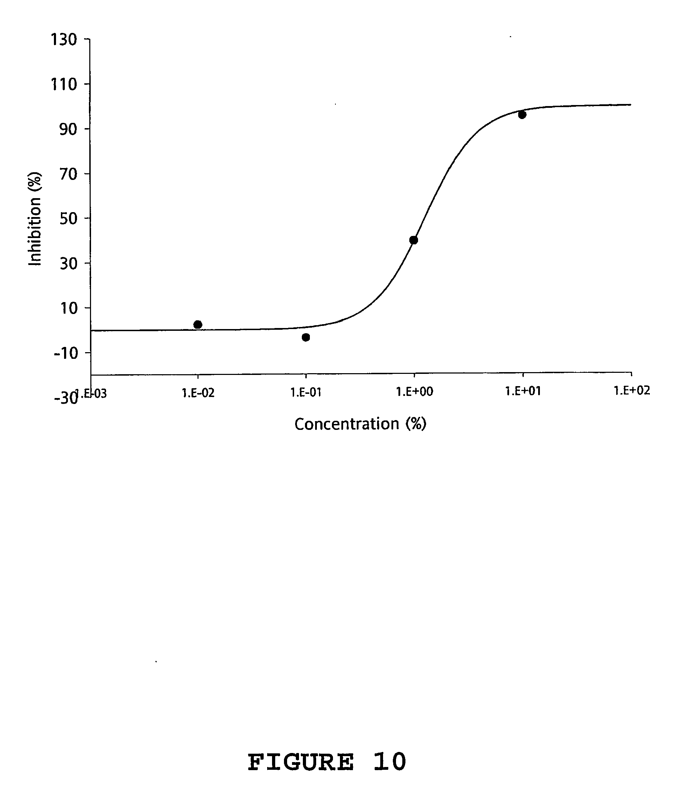

Figure 10 shows a direct measurement of human TACE activity in human recombinant insect Sf21 under the influence of the test sample comprising zinc chloride, glycine and trypsinised protein. This test sample inhibited the TACE activity with an IC₅₀ of 1.3% of the test sample solution. TACE inhibition demonstrates an additional pathway through which the test compositions can reduce an inflammatory response.
Figure 11 shows inhibition of human Caspase 1 *in vitro* with an IC₅₀ of 8.1% of the same test sample solution as in Figure 10.
Figure 12 shows inhibition of human Caspase 3 *in vitro* induced by the same test sample as in Figure 10. The test. sample inhibited caspase 3 with an IC₅₀ of 2.8%.

### DETAILED DESCRIPTION OF PREFERRED EMBODIMENTS

Before describing the present invention in detail, it is to be understood that this invention is not limited to particularly exemplified methods and may, of course, vary. It is also to be understood that the terminology used herein is for the purpose of describing particular embodiments of the invention only, and is not intended to be limiting which will be limited only by the appended claims.

Furthermore, the practice of the present invention employs, unless otherwise indicated, conventional chemistry and pharmacology within the skill of the art. Such techniques are well known to the skilled worker, and are explained fully in the literature. See, eg., Coligan, Dunn, Ploegh, Speicher and Wingfield "Current protocols in Protein Science" (1999) Volume I and II (John Wiley & Sons Inc.); The Merck Index, 12th Edition (1996), Therapeutic Category and Biological Activity Index; and Remington's Pharmaceutical Sciences, 17th Edition, Mack Publishing Company, Easton, Pennsylvania, USA.

It must be noted that as used herein and in the appended claims, the singular forms "a," "an," and "the" include plural reference unless the context clearly dictates otherwise. Thus, for example, a reference to "a metal" includes a plurality of such metals, and a reference to "an isolated protein" is a reference to one or more proteins, and so forth. Unless defined otherwise, all technical and scientific terms used herein have the same meanings as commonly understood by one of ordinary skill in the art to which this invention belongs. Although any materials and methods similar or equivalent to those described herein can be used to practice or test the present invention, the preferred materials and methods are now described.

In its broadest aspect, the present invention provides a composition useful as a treatment agent for tissue disruptions. The term "tissue disruption" refers to abnormal conditions affecting animals, including humans, which can be treated using the agents, therapeutic compositions and wound dressings of the present invention. The term "tissue disruption" refers to wounds.

The injury can be a minor tissue disruption of, for instance, epidermal, dermal, muscular or adipoidal tissue to the air. The term "wounds" includes a puncture wound, an incision, a laceration, a penetrating wound, a perforating wound, a tunnel wound and the like. Wounds also include open wounds that have been sutured or otherwise mechanically closed but have not healed or repaired the break in the skin or oral mucosal layer or of the surface layers of the eye including the conjunctiva and cornea.

The terms "lesion" and "surface lesion" as used herein refer to a circumscribed area of pathologically altered tissue, an injury or wound. Primary lesions are the immediate result of the pathological condition and include, but are not limited to, cuts, abrasions, vesicles, blebs, bullae chancres, pustules, tubercles or any other such condition of the skin or a surface of the mouth, nose, anus or any other orifice of the body of a human or animal, or to the surface layers of the eye including the conjunctiva and cornea, or secondary lesions that later develop from a primary lesion and includes, but is not limited to, fissures and ulcers and other wounds.

The term "tissue disruption management" refers to therapeutic methods that induce and/or promote repair of tissue damage including, but not limited to, arresting tissue damage such as necrotization, promoting tissue growth and repair, reduction or elimination of an established microbial infection of the injury and prevention of new or additional microbial infection or colonization. The term may further include reducing or eliminating the sensation of pain attributable to a wound.

The terms "tissue disruption healing" and "tissue disruption repair" refer to a process involving tissue growth that partially or totally repairs the injury, repairs a breach in the tissue and partially or totally restores the tissue. For example, the barrier properties of the skin, repair of the surface layers of the eye including the conjunctiva and cornea, and breaches in the dermis and/or epidermis, connective tissue, tendons, and ligaments can be repaired or healed.

Without wishing to be bound by any theory or hypothesis, the inventors believe that the composition of the invention is capable of repairing a tissue disruption because of its effect on TNF-α, TNF-αR, TACE, and caspases. Moreover, the inventors believe that the effect on TNF-α, TNF-αR, TACE, and caspases reduces the secondary injury caused by these molecules and modulates apoptosis.

As used herein the term "modulating" includes an increase or a decrease in the activity. Thus the activity of, for example TNF-α, may be higher or lower compared to the level in a subject having TNF-α activity within a normal range.

Generally, the terms "treating," "treatment" and the like are used herein to mean affecting the tissue or cells of an individual to obtain a desired pharmacological and/or physiological effect. The effect is especially therapeutic in terms of a partial or complete cure of a tissue disruption. "Treating" as used herein covers any treatment of a tissue disruption in a vertebrate, a mammal, particularly a human, and includes: (a) inhibiting the tissue disruption, i.e., arresting its development; or (b) relieving or ameliorating the symptoms of the tissue disruption, i.e., cause regression of the symptoms of the tissue disruption.

The terms "subject" or "individual" are used interchangeably herein to refer to any member of the class mammalia, including, without limitation, humans and other mammals such as primates, including non-human primates such as chimpanzees and other apes and monkey species; farm animals such as cattle, sheep, pigs, goats and horses; domestic mammals such as dogs and cats; laboratory animals including rodents such as mice, rats and guinea pigs. The terms do not denote a particular age. Thus, both adult and newborn individuals are intended to be covered.

Thus provided is the treatment of mammals such as humans, as well as those mammals of economical importance and/or social importance to humans, for instance, carnivores other than humans (such as cats and dogs), swine (pigs, hogs, and wild boars), ruminants (such as cattle, oxen, sheep, giraffes, deer, goats, bison, and camels), and horses.

The term "effective amount" refers to that amount which is sufficient to induce tissue disruption healing or repair when administered to a subject; e.g., a tissue disruption healing amount. What constitutes an effective amount, or dose, of the composition of the present invention depends, among other factors, on the body weight of the subject and the degree of injury being treated. Normally an effective dose will be found in the range of about 1 to about 6 mg/kg body weight. For an average 75 kg subject, this range equates to a dose of about 75 to about 450 mg. Proportionately smaller or larger doses can be appropriate for subjects having lesser or greater body weight. Such a dose can be administered as needed, but typically administration 1 to about 4 times per day, in most cases 1 or 2 times a day, provides adequate tissue disruption healing.

The term "plasma" typically refers to the straw-coloured fluid in which the blood cells are suspended. It consists of various inorganic salts of sodium, potassium, calcium, etc, with a high concentration of protein (approximately 70g/1) and a variety of trace elements. The term "serum" refers to the fluid that separates from clotted blood or blood plasma that is allowed to stand. Serum is essentially similar in composition to plasma, but generally lacks fibrinogen and others substances that are used in the coagulation process.

The plasma or serum used in the present invention may be obtained from any animal source. In some embodiments, the plasma and/or serum is isolated from blood taken from an animal selected from the group consisting of human, equine, bovine, ovine, murine, caprine and canine.

In some embodiments, the animal source for the plasma or serum is bovine.

The plasma or serum may be freshly isolated or alternatively lyophilised. In some embodiments, blood is isolated from cattle and the haemoglobin is removed by standard procedures. Plasma may then be mixed with sodium bicarbonate (approx. 20g per litre) and heated to about 80°C. The coagulated plasma protein is then removed and lyophilised by standard procedures for further use.

In some embodiments the lyophilised plasma or serum is resuspended in water (approximately 50g per litre) and mixed with at least one metal.

Various metals and/or metal ions are useful in the composition of the present invention and as such the present invention embraces all such metals or metal ions.

In some embodiments, the metals are selected from the group consisting of nickel, sodium, copper, zinc, cobalt, iron, magnesium, manganese, potassium, silver and mercury.

In some embodiments the composition is prepared by resuspending lyophilised plasma in water (about 50g per litre). The plasma is then exposed to about 1% to about 4% sodium bicarbonate. The term "about" means that the plasma will have a final concentration of 1% give or take around 10% e.g. 0.9% or 1.1%. The "exposing" as used herein refers to the time the plasma and sodium bicarbonate are mixed together or in contact with each other. In some embodiments the plasma is exposed to the sodium bicarbonate for 4 to 5 hours. The temperature of the plasma/bicarbonate mixture may then be gently raised from room temperature to 70-80°C.

In cases where the metals are sufficiently basic or acidic to form stable non-toxic acid or base salts, the use of the metals as salts can be appropriate. Examples of acceptable metal salts include acetate, ascorbate, benzoate, bicarbonate, chloride, citrate, carbonate, α-glycerophosphate, α-ketoglutarate, malonate, methanesulfonate, nitrate, succinate, sulfate, tartarate and tosylate salts.

Metal salts can be obtained using standard procedures well known in the art, for example by reacting a sufficiently basic compound such as an amine with a suitable acid affording a physiologically acceptable anion. Alkali metal (for example, sodium, potassium or lithium) or alkaline earth metal (for example calcium) salts can be made.

In some embodiments, for example, the metal is silver (I), wherein the nitrate salt provides adequate free silver (I) ion to provide the necessary metal requirement. The chloride salt on the other hand provides less silver, being less soluble and with a low dissociation constant and therefore is less useful in the present invention. The skilled artisan will be able to readily determine the suitable salt form of the metal ion that provides the necessary properties for the present invention. Furthermore, the skilled artisan will be aware of the compatibility of the salt forms of the metal(s) and other components of the composition to maintain adequate levels of the metal ion(s).

In some embodiments, the metals used in the composition comprise a mixture of a number of metals. For example, the mixture of metals could consist essentially of NiSO₄.7H₂O, NH₄VO₃, NaF, CuSO₄. 5H₂O, ZnCl₂, (NH₄)₆MO₇O₂₄.4H₂O, COCl₂.6H₂O, FeSO₄.7H₂O, MgSO₄.7H₂O, H₃BO₃, MnCl₂.4H₂O and K₂CrO₄.

Once the metal, metal ion or metal salt thereof has been mixed with the plasma and/or serum, it is heated to at least 50°C. In some embodiments, the mixture is heated to about 65°C.

In some embodiments, one or more proteases selected from the group consisting of trypsin, chymotrypsin, factor Xa, venom-protease, thrombin, plasmin and a serine-protease of the subtilisin family may be added after heating. The protease may be trypsin.

The protease may be added after the metal, metal ion or metal salt is added. Whichever, once the protease has been added the resulting mixture of plasma/serum and protease, with metal, metal ion or metal salt is incubated between about 30°C and 45°C for at least 30 minutes. The mixture is then heated again. The second heating step is carried out between about 80°C and about 150°C. In some embodiments the second heating step is carried out between about 90°C and about 130°C. In other embodiments the second heating step is carried out at about 120°C to produce said tissue disruption treatment mixture.

Once the tissue disruption treatment mixture has been obtained it can be either used directly or fractionated to obtain a more refined tissue disruption treatment active fraction. Techniques for fractionating protein-containing mixtures are well known in the art. See, for example, "Plasma Protein Fractionation" Heide K, Haupt H & Schwick H; in The Plasma Proteins, 2nd Edition Vol 3 (1977) Putnam F. (Ed); US Pat. No. 4,351,710 and US Pat. No. 4,322,275 both entitled "Fractionation of protein mixtures"; US Pat. No. 5,138,034 entitled "Method of fractionating plasma proteins".

As described above, in some embodiments the present invention provides a method of treating tissue disruptions in a subject, the method comprising administering to the subject an effective tissue disruption healing amount of a composition of the present invention.

The method of the invention can be used to treat all types of tissue disruptions as described supra. The method of the invention is useful for treatment of non-human mammalian subjects, including domestic, farm and exotic animals, such as for example dogs horses, zoo animals and the like, but is primarily useful for treatment of human subjects.

Compositions of the present invention can also be used in combination therapies with opioids and other analgesics, including narcotic analgesics, Mu receptor antagonists, Kappa receptor antagonists, non-narcotic (i.e., non-addictive) analgesics, monoamine uptake inhibitors, adenosine regulating agents, cannabinoid derivatives, Substance P antagonists, neurokinin-1 receptor antagonists and sodium channel blockers, among others. In some embodiments the combination therapy comprises a composition useful in methods of the invention with one or more compounds selected from aceclofenac, acemetacin, α-acetamidocaproic acid, acetaminophen, acetaminosalol, acetanilide, acetylsalicylic acid (aspirin), S-adenosylmethionine, alclofenac, alfentanil, allylprodine, alminoprofen, aloxiprin, alphaprodine, aluminum bis(acetylsalicylate), amfenac, aminochlorthenoxazin, 3-amino-4-hydroxybutyric acid, 2-amino-4-picoline, aminopropylon, aminopyrine, amixetrine, ammonium salicylate, ampiroxicam, amtolmetin guacil, anileridine, antipyrine, antipyrine salicylate, antrafenine, apazone, bendazac, benorylate, benoxaprofen, benzpiperylon, benzydamine, benzylmorphine, bermoprofen, bezitramide, α-bisabolol, bromfenac, p-bromoacetanilide, 5-bromosalicylic acid acetate, bromosaligenin, bucetin, bucloxic acid, bucolome, bufexamac, bumadizon, buprenorphine, butacetin, butibufen, butophanol, calcium acetylsalicylate, carbamazepine, carbiphene, carprofen, carsalam, chlorobutanol, chlorthenoxazin, choline salicylate, cinchophen, cinmetacin, ciramadol, clidanac, clometacin, clonitazene, clonixin, clopirac, clove, codeine, codeine methyl bromide, codeine phosphate, codeine sulfate, cropropamide, crotethamide, desomorphine, dexoxadrol, dextromoramide, dezocine, diampromide, diclofenac sodium, difenamizole, difenpiramide, diflunisal, dihydrocodeine, dihydrocodeinone enol acetate, dihydromorphine, dihydroxyaluminum acetylsalicylate, dimenoxadol, dimepheptanol, dimethylthiambutene, dioxaphetyl butyrate, dipipanone, diprocetyl, dipyrone, ditazol, droxicam, emorfazone, enfenamic acid, epirizole, eptazocine, etersalate, ethenzamide, ethoheptazine, ethoxazene, ethylmethylthiambutene, ethylmorphine, etodolac, etofenamate, etonitazene, eugenol, felbinac, fenbufen, fenclozic acid, fendosal, fenoprofen, fentanyl, fentiazac, fepradinol, feprazone, floctafenine, flufenamic acid, flunoxaprofen, fluoresone, flupirtine, fluproquazone, flurbiprofen, fosfosal, gentisic acid, glafenine, glucametacin, glycol salicylate, guaiazulene, hydrocodone, hydromorphone, hydroxypethidine, ibufenac, ibuprofen, ibuproxam, imidazole salicylate, indomethacin, indoprofen, isofezolac, isoladol, isomethadone, isonixin, isoxepac, isoxicam, ketobemidone, ketoprofen, ketorolac, p-lactophenetide, lefetamine, levorphanol, lofentanil, lonazolac, lomoxicam, loxoprofen, lysine acetylsalicylate, magnesium acetylsalicylate, meclofenamic acid, mefenamic acid, meperidine, meptazinol, mesalamine, metazocine, methadone hydrochloride, methotrimeprazine, metiazinic acid, metofoline, metopon, mofebutazone, mofezolac, morazone, morphine, morphine hydrochloride, morphine sulfate, morpholine salicylate, myrophine, nabumetone, nalbuphine, 1-naphthyl salicylate, naproxen, narceine, nefopam, nicomorphine, nifenazone, niflumic acid, nimesulide, 5'-nitro-2'-propoxyacetanilide, norlevorphanol, normethadone, normorphine, norpipanone, olsalazine, opium, oxaceprol, oxametacine, oxaprozin, oxycodone, oxymorphone, oxyphenbutazone, papaveretum, paranyline, parsalmide, pentazocine, perisoxal, phenacetin, phenadoxone, phenazocine, phenazopyridine hydrochloride, phenocoll, phenoperidine, phenopyrazone, phenyl acetylsalicylate, phenylbutazone, phenyl salicylate, phenyramidol, piketoprofen, piminodine, pipebuzone, piperylone, piprofen, pirazolac, piritramide, piroxicam, pranoprofen, proglumetacin, proheptazine, promedol, propacetamol, propiram, propoxyphene, propyphenazone, proquazone, protizinic acid, ramifenazone, remifentanil, rimazolium metilsulfate, salacetamide, salicin, salicylamide, salicylamide o-acetic acid, salicylsulfuric acid, salsalte, salverine, simetride, sodium salicylate, sufentanil, sulfasalazine, sulindac, superoxide dismutase, suprofen, suxibuzone, talniflumate, tenidap, tenoxicam, terofenamate, tetrandrine, thiazolinobutazone, tiaprofenic acid, tiaramide, tilidine, tinoridine, tolfenamic acid, tolmetin, tramadol, tropesin, viminol, xenbucin, ximoprofen, zaltoprofen and zomepirac (see The Merck Index, 12th Edition (1996), Therapeutic Category and Biological Activity Index, lists therein headed "Analgesic", "Anti-inflammatory" and "Antipyretic").

Still other suitable formulations for use in the present invention can be found in Remington's Pharmaceutical Sciences, Mace Publishing Company, Philadelphia, Pa. 17th ed. (1985).

The terms "administration", "administering", and "administered" are used herein interchangeably. The tissue disruption healing composition of the present invention is administered topically in dosage unit formulations containing conventional non-toxic pharmaceutically acceptable carriers, adjuvants, and vehicles. In some embodiments the tissue disruption healing composition of the present invention is administered together with a pharmaceutically acceptable carrier or diluent compatible with the composition. In preparing such composition, any conventional pharmaceutically acceptable carrier can be utilised.

The carrier material can be an organic or inorganic inert carrier material suitable for oral administration. Suitable carriers include water, gelatin, gum arabic, lactose, starch, magnesium stearate, talc, vegetable oils, polyalkylene-glycols, petroleum jelly and the like. Furthermore, the pharmaceutically active preparations may contain other pharmaceutically active agents. Additionally, additives such as flavouring agents, preservatives, stabilisers, emulsifying agents, buffers and the like may be added in accordance with accepted practices of pharmaceutical compounding.

For topical administration to the skin or mucous membrane the aforementioned tissue disruption healing composition of the present invention may be prepared as an ointment, tincture, cream, gel, solution, lotion, spray, aerosol, dry powder for inhalation, suspension, and the like. In fact, any conventional methods of preparing topical compositions can be utilised in this invention. Among the preferred methods of applying the tissue disruption healing composition of the present invention is in the form of an ointment, gel, cream, lotion, spray, aerosol, or dry powder. A pharmaceutical preparation for topical administration to the skin can be prepared by mixing the tissue disruption healing composition of the present invention with non-toxic, therapeutically inert, solid or liquid carriers customarily used in such preparation. These preparations generally contain 0.01 to 5.0 percent by weight, preferably 0.1 to 1.0 percent by weight, of the tissue disruption healing composition of the present invention, based on the total weight of the composition.

In preparing the topical preparations described above, additives such as preservatives, thickeners, perfumes and the like conventional in the art of pharmaceutical compounding of topical preparation can be used. In addition, conventional antioxidants or mixtures of conventional antioxidants can be incorporated into the topical preparations containing the afore-mentioned active agent. Among the conventional antioxidants which can be utilised in these preparations are included N-methyl-α-tocopherolamine, tocopherols, butylated hydroxyanisole, butylated hydroxytoluene, ethoxyquin and the like. Cream-base pharmaceutical formulations containing the antigen preparation, used in accordance with this invention, are composed of aqueous emulsions containing a fatty acid alcohol, semi-solid petroleum hydrocarbon, ethylene glycol and an emulsifying agent.

Ointment formulations containing the tissue disruption healing composition of the present invention may comprise admixtures of a semi-solid petroleum hydrocarbon with a solvent dispersion of the tissue disruption healing composition. Cream compositions containing the tissue disruption healing composition of this invention may comprise emulsions formed from a water phase of a humectant, a viscosity stabiliser and water, an oil phase of a fatty acid alcohol, a semi-solid petroleum hydrocarbon and an emulsifying agent and a phase containing tissue disruption healing composition dispersed in an aqueous stabiliser-buffer solution. Stabilisers may be added to the topical preparation. Any conventional stabiliser can be utilised in accordance with this invention. In the oil phase, fatty acid alcohol components function as a stabiliser. These fatty acid alcohol components are derived from the reduction of a long-chain saturated fatty acid containing at least 14 carbon atoms.

Formulations for aerosols are described in Drugs and Pharmaceutical Sciences, Marcel Dekker, New York, 72: 547-574 (1996). Furthermore, the tissue disruption healing composition of the present invention can be delivered by dry powder inhalation. Such formulations and devices are described in Pharmaceutical Technology, June 1997, pp.117-125.

Depending upon the mode or type of administration and the severity of the tissue disruption, the treatment regime will vary.

In some embodiments, the compositions of the present invention are used directly as wound dressings. For example, as described supra, the resultant compositions can be used as a wound dressings directly. However, in further embodiments the compositions of the present invention can be incorporated into "traditional" wound dressings such as plasters, bandages, gauze or pads.

In use, the wound dressings of the present invention are preferably used as the primary dressing placed in direct contact with the wound bed, or as near as practical against the wound bed. The dressings may serve as a packing material and, if required, may be secured into position with any suitable secondary wound dressing such as a wrap, tape, gauze, or pad. The dressings are temporary, however, and are not intended for permanent incorporation into the healed tissues. When necessary, the wound dressings are changed by first removing any overdressing material and then removing the dressing, whereby any accumulated necrotic tissue and exudate is lifted away. The wound dressing of the present invention may be replaced by a fresh dressing or other suitable wound covering.

The dressings may be placed in their entirety into a wound. The dressings of the present invention may be cut, shaped and modified to accommodate numerous uses and applications.

A further use for the therapeutic composition of the present invention is in the delivery of therapeutically active agents including in any of the aforementioned applications. Therapeutically active agents may participate in, and improve, the healing process, and may include anti-microbial agents, including but not limited to anti-fungal agents, anti-bacterial agents, anti-viral agents and anti-parasitic agents, growth factors, angiogenic factors, anaesthetics, mucopolysaccharides, metals and other healing agents.

Examples of anti-microbial agents that can be used in the present invention include, but are not limited to, isoniazid, ethambutol, pyrazinamnide, streptomycin, clofazimine, rifabutin, fluoroquinolones, ofloxacin, sparfloxacin, rifampin, azithromycin, clarithromycin, dapsone, tetracycline, erythromycin, ciprofloxacin, doxycycline, ampicillin, amphotericin B, ketoconazole, fluconazole, pyrimethamine, sulfadiazine, clindamycin, lincomycin, pentamidine, atovaquone, paromomycin, diclazaril, acyclovir, trifluorouridine, foscarnet, penicillin, gentamicin, ganciclovir, iatroconazole, miconazole, Zn-pyrithione, heavy metals including, but not limited to, gold, platinum, silver, zinc and copper, and their combined forms including, salts, such as chloride, bromide, iodide and periodate, and complexes with carriers, and other forms.

Growth factor agents that may be incorporated into the tissue disruption/wound dressing devices of the present invention include, but are not limited to, basic fibroblast growth factor (bFGF), acidic fibroblast growth factor (aFGF), nerve growth factor (NGF), epidermal growth factor (EGF), insulin-like growth factors 1 and 2, (IGF-1 and IGF-2), platelet derived growth factor (PDGF), tumor angiogenesis factor (TAF), vascular endothelial growth factor (VEGF), corticotropin releasing factor (CRF), transforming growth factors α and β (TGF-α and TGF-β) interleukin-8 (IL-8), granulocyte-macrophage colony stimulating factor (GM-CSF), the interleukins, and the interferons.

Other agents that may be incorporated into the dressings of the present invention are acid mucopolysaccharides including, but not limited to, heparin, heparin sulfate, heparinoids, dermatan sulfate, pentosan polysulfate, cellulose, agarose, chitin, dextran, carrageenin, linoleic acid, and allantoin.

In some particularly preferred embodiments, the therapeutic composition of the present invention is admixed with coagulant agents such as Factor Xa.

The therapeutically active agents may be bound, either physically or chemically, to the therapeutic composition by methods well known in the art.

Throughout the specification, the word "comprise" and variations of the word, such as "comprising" and "comprises", means "including but not limited to" and is not intended to exclude other additives, components, integers or steps. By "consisting of" is meant including, and limited to, whatever follows the phrase "consisting of". Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that no other elements are optional and may or may not be present depending upon whether or not they affect the activity or action of the listed elements.

The invention will now be further described by way of reference only to the following non-limiting examples. It should be understood, however, that the examples following are illustrative only, and should not be taken in any way as a restriction on the generality of the invention described above. In particular, while the invention is described in detail in relation to the use of specific animal plasma and metals, it will be clearly understood that the findings herein are not limited to these ingredients.

### EXAMPLE 1 PREPARATION OF TISSUE DISRUPTION TREATMENT

### COMPOSITION

200 litres of sterile cattle blood was centrifuged at 1000 - 1300 x g for 10 minutes and the haemoglobin was removed from the plasma. After centrifugation approximately 100 litres of plasma was gained, and transferred into a dish, suitable for heating and continuous mixing. 2kg sodium bicarbonate (NaHCO₃) was added to the plasma and mixed until the NaHCO₃ dissolved, then the solution was heated to 80°C. Denatured plasma protein was then recovered and placed on filter paper to dry. The solid sediment was then pressed to produce a 60kg solid plasma-protein "block" which was then lyophilised by standard procedures. After this process the plasma-protein weighed approximately 8kg and was used in the preparation of the tissue disruption treatment composition as described below.

A solution was then prepared comprising 152 litres of water, 8kg dried plasma-protein as prepared above and 200ml of a metal-containing solution. The constituents of the metal-containing solution are shown in Table 1.

**TABLE 1**

| METAL-CONTAINING SOLUTION | |
|---|---|
| Ni SO₄ 7 h₂O | 10.4g/l |
| NH₄VO₃ | 1.2g/l |
| Na F | 24.0g/l |
| Cu SO₄ 5H₂O | 20.0g/l |
| ZN Cl₂ | 47.0g/l |
| (NH₄) 6 MO₇O₂₄ 4H₂O | 7.0g/l |
| CO Cl₂ 6H₂O | 20.0g/l |
| Fe SO₄ 7H₂O | 100.0g/l |
| MgSO₄ 7H₂O | 80.0g/l |
| H₃BO₃ | 23.0g/l |
| Glucose | 50.0g/l |
| Mn Cl₂ 4H₂O | 36.4g/l |
| K₂CrO₄ | 1.0g/l |
| Glycine | 75.0g/l |
| Citric Acid | 20.0g/l |

Made up in a 200ml solution with water, which was then stirred for at least 20 minutes.

The mixture was then heated to 120°C and maintained at this temperature for two hours with constant mixing. During this time the plasma-protein dissolved and was sterilized. The resulting material was then held at a temperature of about 35°C and 0.125g/l of trypsin was added. The material was then allowed to incubate for approximately 2 hours. The digested material was then autoclaved and cooled to produce the tissue disruption treatment composition of the present invention.

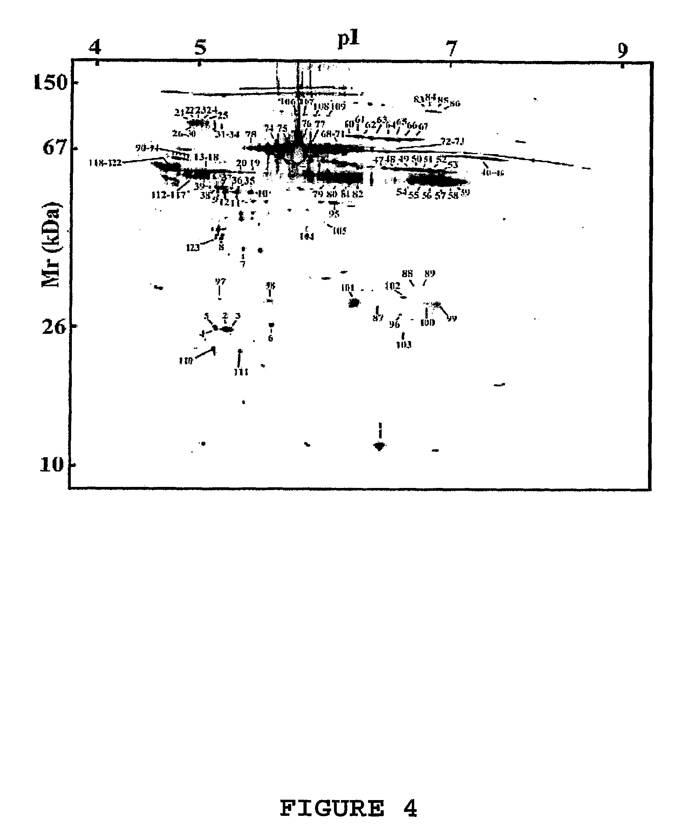

Figures 3 and 4 show the soluble plasma protein fragments obtained by this method. In Figure 3, the major protein bands apparent in untreated plasma separated by SDS-PAGE are 50-80 kDa in size (Lane 5) Proteomic analysis of these bands identified them as consisting mainly of albumin, immunoglobulins, fibrinogen and transferrin (Figure 4).

In contrast, the plasma protein before protease treatment consists mainly of polypeptides smaller than 50 kDa (Figure 3. Lane 2), while after trypsinisation and addition of metals the soluble plasma protein fragments are reduced to molecular weights of less than 25 kDa (10-20% tricine gradient gel, Figure 3. Lane 3).

### EXAMPLE 2 MANUFACTURE OF A TOPICAL TREATMENT COMPOSITION

A composition comprising the ingredients shown in Table 2 were mixed at 75-80°C in a 250 litre vacuum homogenizer equipped with anchor and turbo mixers. Then the ingredients shown in Table 3 were added and the mixing was continued at 80-83°C for 10 minutes with the aid of the turbo mixer.

A slow cooling process was then carried out using the anchor mixer. When the material reached 60°C, the vacuum was switched on until the end of the cooling.

At 40-45°C the ingredients shown in Table 4 were added and mixed for 10 minutes. Mixing with the anchor mixer was continued until the mixture reached 25°C.

After a standing period of approximately 24 hours, the tissue disruption treatment composition was ready for use.

**TABLE 2**

| Item No. | Amount Per Kg | Ingredients |
|---|---|---|
| 1 | 20g | Liposorb S20 (Tween 60) |
| 2 | 20g | Cremaphor A6 |
| 3 | 10g | Hydromyristenol |
| 4 | 40g | Cetyl alcohol |
| 5 | 70g | Corn Oil (Cold Pressed) |
| 6 | 30g | Wheat Germ Oil |
| 7 | 0.24g | Carrot Oil |
| 8 | 50g | Isopropyl Myristate |
| 9 | 0.2g | Butylated Hydroxytoluene B.P. |
| 10 | 3g | Phenonip |

**TABLE 3**

| | | |
|---|---|---|
| 11 | 400g | Plasma protein from Example 1 |
| 12 | 15g | Propylene Glycol B.P. |
| 13 | 15g | Hygroplex HHG |
| 14 | 2g | Allantoin |
| 15 | 208g | Purified Water B.P. |
| 16 | 10g | Germaben II |
| 17 | 4g | Veegum |
| 18 | 100g | Purified Water B.P. |
| 19 | 0.04ml | Potassium Bromide 50g/l |
| 20 | 30.7mg | Sodium Sulphide |
| 21 | 0.04ml | Potassium Iodide 25g/l |

**TABLE 4**

| | | |
|---|---|---|
| 22 | 1.4g | Chammomile Fragrence |

### Methodology

1). Add items 1 to 10 in a 250 litre steam pan and heat 75°C;
2). Boil items 15 and 18 in the 150 litre pan and transfer 13 litres to the 50 litre pan and add Veegum and mix until homogeneous;
3). Add item 14 to the remainder of the Purified Water B.P. in the 150 litre steam pan at above 90°C and mix. When dissolved add the items 12, 13 and 16 and maintain temperature at 75°C with continual mixing;
4). Add the water phase (step 5) to the oil phase (step 3) and mix using a short shaft air mixer. Then add step 4 to this using a plastic sieve to ensure that no lumps are incorporated;
5). Add plasma protein from Example 1 and emulsify for 20 minutes, then continue stirring whilst water cooling to 40°C;
6). Add items 19 to 21 allowing a few minutes in between each addition whilst mixing. Cool to below 30°C.

### EXAMPLE 3 CLINICAL TRIAL ON TOPICAL SOFT TISSUE INJURY TREATMENT

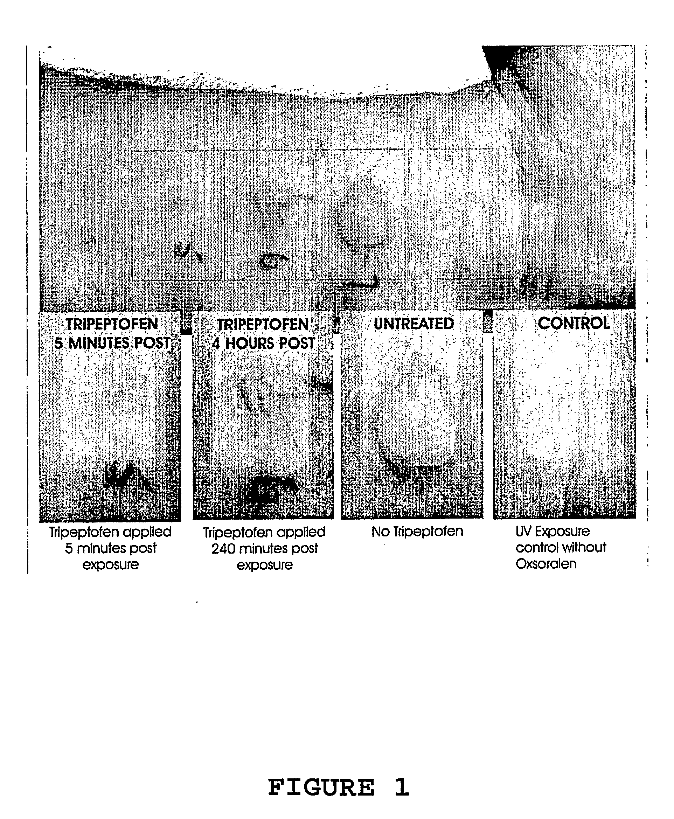

As shown in Figure 1, a patient was exposed to UV light at 800mJ for 10 minutes. Topical application of a 1% Oxsoralen (C₁₂H₈O₄) lotion was used on regions 5, 6 and 7 as a photo sensitizer. Region 8 remained an exposure control. Region 7 received no therapeutic treatment post exposure. Region 6 received topical treatment with the tissue disruption treatment composition described in Example 2 above after 240 minutes post-exposure, while region 5 received a similar amount of tissue disruption treatment composition 5 minutes post-exposure.

The pictures shown in Figure 1 were taken 24 hours after exposure. As can be clearly seen there is a vast difference in both treated and untreated areas and between 5 minute post-exposure treatment and 240 minute post-exposure treatment with the tissue disruption treatment composition. As can be seen, control region 7 has a large fluid filled lesion after 24 hours. The application of the tissue disruption treatment composition after 240 minute post-exposure (Region 6) reduced the severity of the lesion produced. The application of the tissue disruption treatment composition to region 5 within 5 minutes of exposure appeared to protect the skin from lesion.

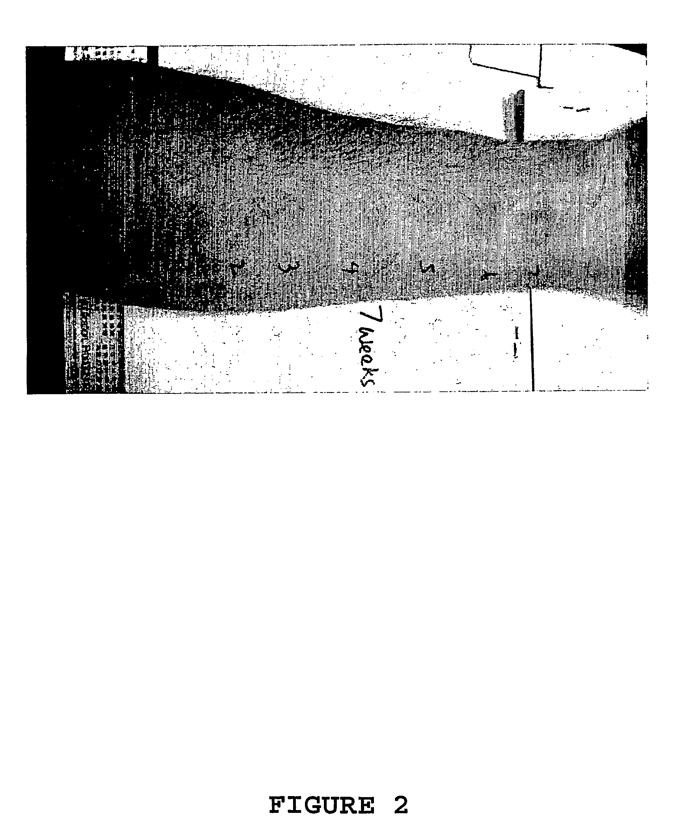

Figure 2 shows the above regions 7 weeks post exposure. Apart from regions 1, 6 and 7 all of the regions had returned to normal skin.

### EXAMPLE 4 TEST OF COMPOSITION ON TNF-α PRODUCTION BY LPS-STIMULATED HUMAN MONOCYTES

The soluble plasma test composition of Example 1 was assayed for its ability to affect TNF-α levels. TNF-α is a cytokine known to be involved in healing of tissue disruptions, thus if the TNF-α levels were reduced in the assay then this demonstrated that the soluble plasma test composition had activity in healing tissue disruptions. In the present experiment the aim was to demonstrate that the soluble plasma test composition was capable of regulating or affecting the presence of TNF-α.

*Human Monocyte Isolation:* Buffy coats were separated by centrifugation from citrate-anti-coagulated whole human blood collected from donors at The Australian Red Cross Blood Service. Buffy coats were diluted in RPMI 1640 medium (Life Technologies), and peripheral blood mononuclear cells (PBMC) were isolated by density gradient centrifugation with Ficoll-Hypaque (Amersham Pharmacia Biotech). Monocytes were isolated from the PBMC by counter-current elutriation centrifugation (Lund et al., 2000). Monocyte purity was determined by CD14 immunophenotyping. Monocytes isolated by this method were typically 80-90% pure.

Monocytes were cultured overnight in RPMI supplemented with 10% Fetal Calf Serum (FCS) and 25ng/mL Macrophage Colony Stimulating Factor (M-CSF)] in the presence of 95% air plus 5% CO₂ at 37°C.

Next day monocytes were counted and 5 x 10⁵ cells per well were placed into wells of a 96 well tissue culture plate. The volume was made up to 500µL per well and then the cells were stimulated with 500ng/mL lipopolysaccharide (LPS) from *E. coli* 0111:B4 (Sigma-Aldrich) in the presence of 1% Fetal Calf Serum (FCS), with varying concentrations of test composition for 24 hours.

TNF-α levels in the culture supernatants were measured by ELISA Opti EIA, BD Bioscience following the manufacturer's instructions. In short, following 24 hour incubation, culture media samples were collected and cells and particulates were removed by centrifugation. Clarified supernatants were stored at -70°C and assayed in batch. The concentration of TNF-α in the culture supernatants was measured using a commercial cytokine ELISA set (BD Biosciences) according to the manufacturer's instructions. TNF-α concentrations in the culture media were derived from a standard curve (125-8000pg/ml).

The detection system selected for these ELISAs was time-resolved fluorescence (TRF) with europium. TRF has been used in many biological systems as a means to reduce background fluorescence and increase sensitivity. Lanthanides such as europium exhibit a large Stokes shift, with excitation occurring by absorbance of UV light with emission wavelengths greater than 500nm. Europium exhibits excitation at 340nm and emission at 615nm.

In the present experiment, the soluble plasma test composition of Example 1 was mixed with zinc chloride (0.006157 g/L) and glycine (0.1965 g/L).

The concentrations of the test composition used were 40% (200µL); 20% (100µL); 10% (50µL); and 0%. The control was LPS (500ng/mL) and there were 3 repeats.

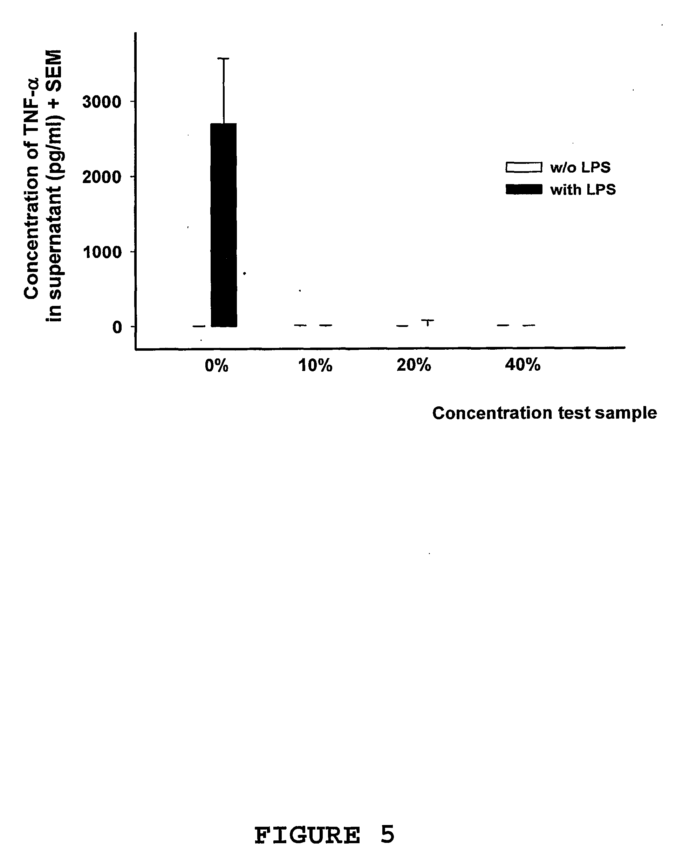

*Data Analysis:* Results were collated and mean and standard error of the mean (SEM) were calculated for each experimental condition. Monocyte TNF-α secretion into culture media was expressed as pg/ml. The effects of LPS and test composition treatments on the concentration of TNF-α in the supernatant were assessed by a Treat by LPS (with the levels LPS and no LPS) analysis of variance (ANOVA), supplemented with Fisher's least significant difference (LSD) post hoc comparisons. The factor Treat had levels no treat, 10, 20, and 40% of the test composition. A difference between groups was considered as statistically reliable if the associated probability (p value) was below 0.05. Table 1 together with Figure 5 shows the results.

**TABLE 1**

| Av pg/mL | w/o LPS | with LPS | | SEM | w/o LPS | with LPS |
|---|---|---|---|---|---|---|
| Test 40% | 0 | 0 | | 40% | 3.28 | 1.25 |
| Test 20% | 0 | 0 | | 20% | 2.17 | 72.18 |
| Test 10% | 0 | 0 | | 10% | 11.01 | 12.88 |
| Test 0% | 0 | 2700.23 | | 0% | 3.72 | 861.07 |

| | | | | | | |
|---|---|---|---|---|---|---|
| SEM: Standard Error of the Mean | | | | | | |

Statistical analysis: The LPS challenge produced a large quantity of TNF-α secretion in untreated cells at 24 hours after incubation (LPS: F_{1,16} = 10.17, p < 0.01). The treatments affected the TNF-α response to the LPS challenge (Treat: F_{3,16} = 9.69, p < 0.001; Treat by LPS: F_{3,16} = 9.19, p < 0.001). Post hoc comparisons revealed that LPS-challenged, test sample treated cells produced less TNF-α secretion than the LPS-challenged, untreated cells. Unchallenged cells did not produce measurable amounts of TNF-α with any of the treatments. Treatment with the test sample resulted in suppression of TNF-α to a level indistinguishable from unchallenged cells.

The conclusions that can be drawn from the results are that the soluble plasma test composition decreases LPS-induced TNF-alpha secretion in human monocytes, indicating efficacy in promoting repair of tissue disruption.

### EXAMPLE 5 EFFECT OF SOLUBLE PLASMA TEST COMPOSITION ON TNF-α PRODUCTION BY LPS-STIMULATED HUMAN MONOCYTES

This experiment was essentially a repeat of the experiment described in Example 4, with the only difference being the soluble plasma test composition in Example 1 was mixed with a metal-containing solution containing only copper sulphate (0.00262 g/L).

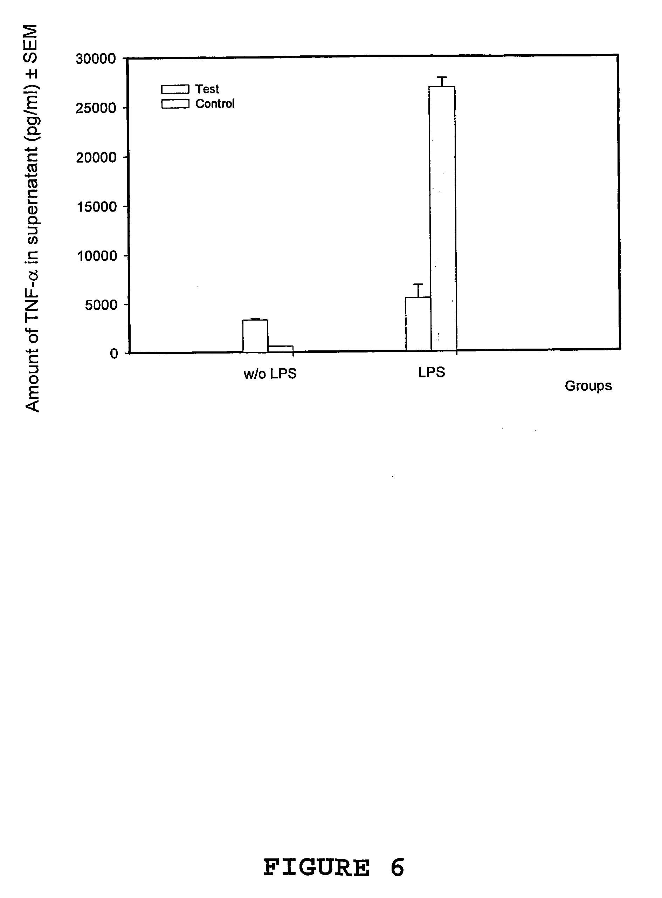

Table 2 and Figure 6 show the results.

The conclusions that can be drawn from these results are that the soluble plasma test composition inhibits the inflammatory response of human monocytes to an LPS challenge.

**TABLE 2**

| Av pg/mL | Ctrl | LPS | | SEM | Ctrl | LPS |
|---|---|---|---|---|---|---|
| Test | 3310.25 | 5508.58 | | Test | 138.24 | 1321.58 |
| Ctrl | 612.87 | 26873.00 | | Ctrl | 6.25 | 932.93 |

### EXAMPLE 6 TEST OF LOWER CONCENTRATION OF SOLUBLE PLASMA TEST COMPOSITION ON TNF-α PRODUCTION BY LPS-STIMULATED HUMAN MONOCYTES

Test of the soluble plasma test composition used in Example 4 on TNF-α production by LPS-stimulated human monocytes was undertaken, but at lower concentrations.

All other experimental procedures were identical to those used in Example 4.

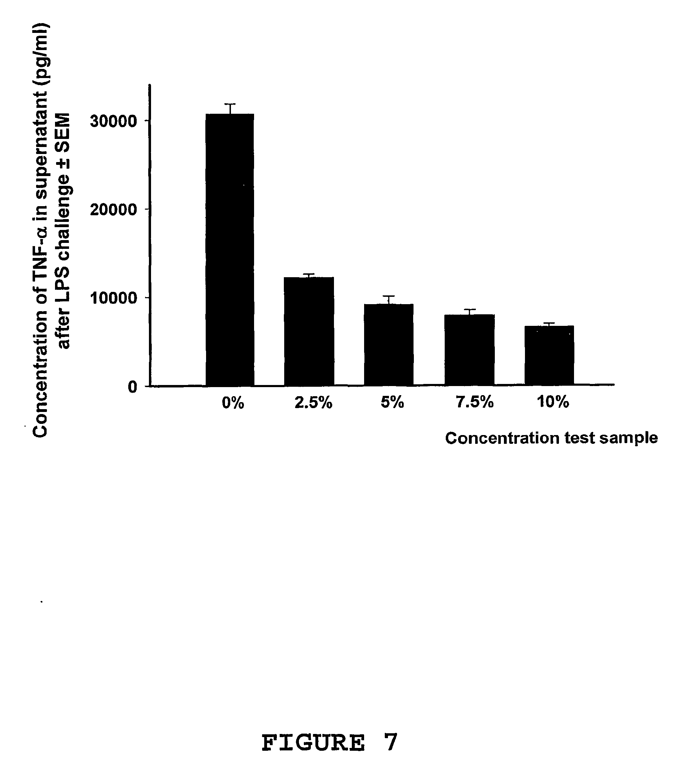

Table 3 and Figure 7 show the results.

The conclusions that can be drawn from these results are that the tissue-disruption repair effect of the soluble plasma test composition is dependent on the dosage, further supporting the outcomes of Example 4, i.e. that LPS-induced TNF-alpha secretion is inhibited by the test composition.

**TABLE 3**

| Groups | Average (pg/mL) | SEM |
|---|---|---|
| Test 10% | 6627.10 | 363.07 |
| Test 7.5% | 7953.37 | 579.38 |
| Test 5.0% | 9138.62 | 945.71 |
| Test 2.5% | 12211.49 | 412.64 |
| Ctrl | 30723.52 | 1140.03 |

### EXAMPLE 7 TITRATION OF THE EFFECT OF DIFFERENT CONCENTRATIONS OF SOLUBLE PLASMA TEST COMPOSITION

Elutriated monocytes were incubated for 24h with a checker-board pattern of soluble plasma test composition (10%, 5%, 2.5% & 0%) as used in Example 4 with various concentrations of FCS (10%, 5%, 1% and 0%). TNF-α was measured by ELISA in the culture supernatants as described above in Example 2.

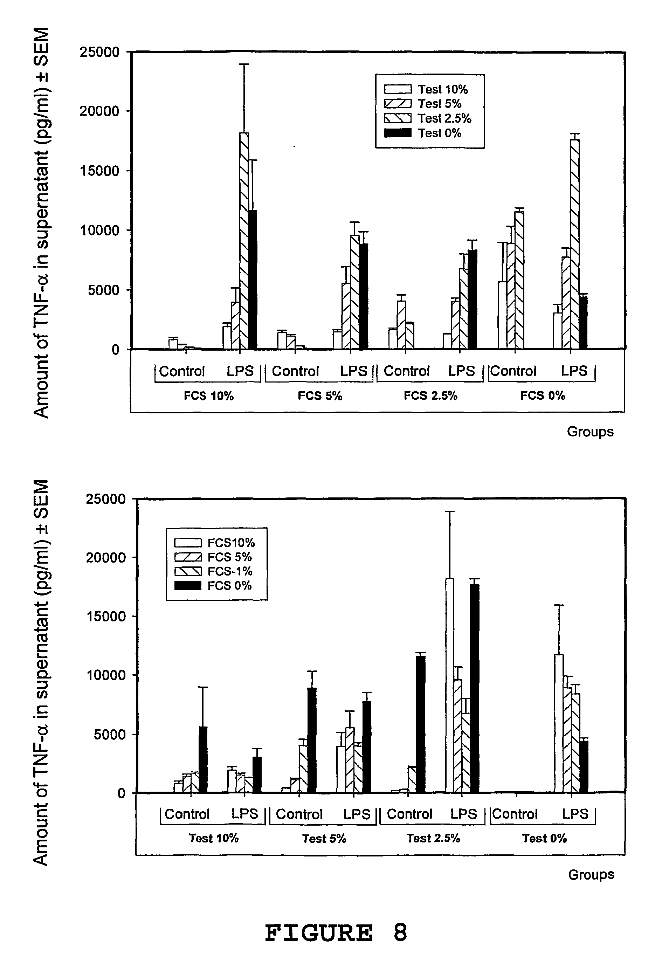

Results are shown in Table 4 and Figure 8.

The conclusions that can be drawn from these results are that the soluble plasma test composition does not compete in inhibiting TNF-alpha secretion with the FCS.

**TABLE 4**

| Av pg/mL | Control | LPS | | SEM | Control | LPS |
|---|---|---|---|---|---|---|
| Test 10%/FCS-10% | 823.17 | 1919.78 | | Test 10%/FCS-10% | 205.20 | 300.87 |
| Test 10%/FCS-5% | 1417.08 | 1484.48 | | Test 10%/FCS-5% | 184.84 | 178.36 |
| Test 10%/FCS-1% | 1647.46 | 1273.60 | | Test 10%/FCS-1% | 125.46 | 20.42 |
| Test 10%/FCS-0% | 5667.25 | 3059.38 | | Test 10%/FCS-0% | 3320.00 | 719.32 |
| | | | | | | |
| Test 5%/FCS-10% | 402.75 | 3961.06 | | Test 5%/FCS-10% | 40.86 | 1191.91 |
| Test 5%/FCS-5% | 1123.04 | 5544.84 | | Test 5%/FCS-5% | 134.77 | 1394.17 |
| Test 5%/FCS-1% | 4037.54 | 4020.51 | | Test 5%/FCS-1% | 535.61 | 271.60 |
| Test 5%/FCS-0% | 8899.82 | 7748.21 | | Test 5%/FCS-0% | 1411.18 | 774.46 |
| | | | | | | |
| Test 2.5%/FCS-10% | 172.99 | 18144.74 | | Test 2.5%/FCS-10% | 12.95 | 5740.39 |
| | 289.20 | 9552.10 | | Test 2.5%/FCS-5% | 5.41 | 1102.56 |
| Test 2.5%/FCS-1% | 2139.26 | 6752.15 | | Test 2.5%/FCS-1% | 117.41 | 1254.98 |
| Test 2.5%/FCS-0% | 11552.74 | 17645.83 | | Test 2.5%/FCS-0% | 328.99 | 504.27 |
| | | | | | | |
| Test 0%/FCS-10% | 93.50 | 11675.28 | | Test 0%/FCS-10% | 5.63 | 4217.03 |
| Test 0%/FCS-5% | 99.80 | 8879.63 | | Test 0%/FCS-5% | 7.12 | 989.86 |
| Test 0%/FCS-1% | 101.16 | 8374.13 | | Test 0%/FCS-1% | 1.85 | 779.93 |
| Test 0%/FCS-0% | 104.32 | 4422.27 | | Test 0%/FCS-0% | 2.36 | 251.71 |

### EXAMPLE 8 AQUEOUS NON-RADIOACTIVE PROLIFERATION ASSAY

In order to show that the soluble plasma test composition of the present invention does not disturb the metabolism of cells *in vitro* and, thus, the TNF-α suppressive effect was not due to a metabolism problem of the cells a non-radioactive proliferation assay was conducted.

The specific assay used was the CellTiter 96® AQᵤₑₒᵤₛ Non-Radioactive Cell Proliferation Assay from Promega. This method is a non-radioactive alternative to the [³H] thymidine incorporation cell proliferation assay. Essentially, the manufacturer's instructions were followed, but briefly, 100µL of 5 x 10⁶ K562 (human chronic myelogenous leukaemia) cells in RPMI supplemented with 5% fetal bovine serum (FBS) were added to the wells of a 96-well plate. Cells were then incubated for 20 hours at 37°C in a humidified, 5% CO₂ atmosphere. The medium was then exchanged and allowed to equilibrate for 1 hour, then 20µL of a solution comprising (3-(4,5-dimethylthiazol-2-yl)-5-(3-carboxymethoxyphenyl)-2-(4-sulfophenyl)-2H-tetrazolium, inner salt; (MTS) and phenazine methosulfate (PMS) was added to each well. A 0hr absorbance reading at 490nm was taken immediately and then absorbance was measured every hour thereafter. Readings at 21 and 45 hours after the addition of the MTS/PMS solution were also taken.

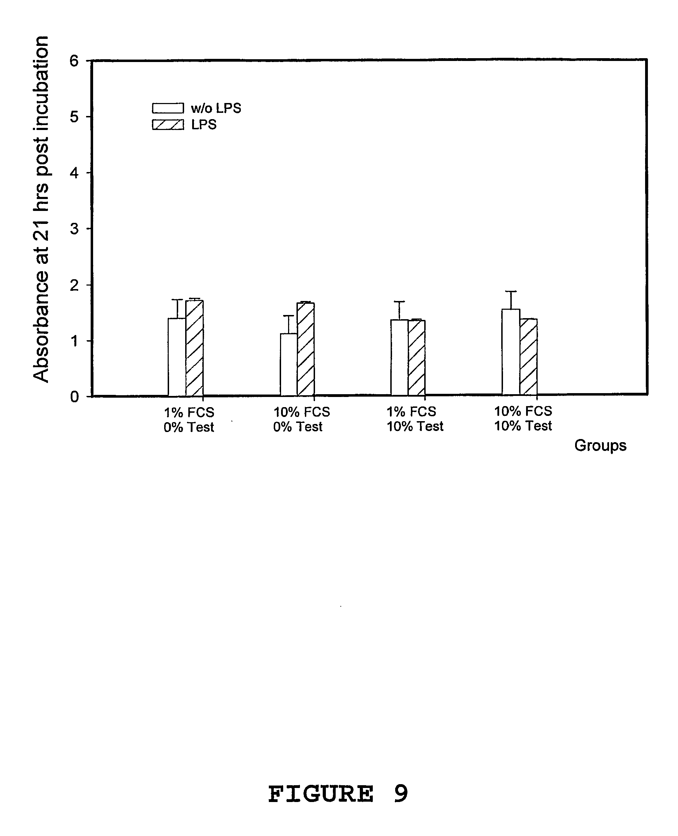

It can be seen from Figure 9 that these cells do not proliferate. The dye wears off with a higher metabolism, which is reflected in higher absorbance (y-axis). The data from TL-treated + LPS challenged cells shows that the test samples were slightly less metabolically active than the controls, but at the same time TNF-α secretion was suppressed. These data are not totally unexpected as the need for a higher metabolism when compared with the untreated + LPS-challenged cells would be less for these cells. Non-LPS-challenged cells do not differ in metabolism, whether treated with the soluble plasma test composition or not.

From these data it can be concluded that the inhibition of TNF-α secretion seen in Examples 4, 5, and 6, was not due to a reduction in metabolic functioning of the cells.

### EXAMPLE 9 INHIBITION OF TACE

TNF-α is initially expressed on the cell surface as a 26-kDa, type II trans-membrane pro-form. The membrane-bound pro-TNF-α can then be cleaved between Ala-76 and Val-77 by a Zn-metalloprotease, TNF-α converting enzyme (TACE), resulting in the formation of the 17-kDa, mature, soluble cytokine.

TACE belongs to the family of metalloprotease disintegrins (also known as ADAM or MDC family), which are modular transmembrane proteins with a Zinc-dependent catalytic domain. Metalloprotease disintegrins are synthesized as inactive precursors containing a prodomain that blocks the activity of the catalytic domain. TACE is the predominant protease responsible for the generation of soluble TNF-α. T cells derived from TACE^{ΔZn/ΔZn} knockout mice have a 90% reduction in their ability to process pro-TNF-α. Levels of TACE protein and its enzymatic activity in the synovial tissue of patients with RA are significantly higher than those of patients with osteoarthritis. Therefore, TACE inhibitors, which inhibit the processing of pro-TNF-α on the plasma membrane, represent an appealing alternative to the neutralization of TNF-α by biological agents.

TACE is also required for the activation of the receptor for the epidermal growth factor (EGFR) in vivo and for the development of tumors in nude mice, indicating a crucial role of TACE in tumorigenesis. In agreement with this view, TACE is dramatically over-expressed in the majority of mammary tumors analyzed. Collectively, this evidence points to TACE as a promising target of anti-tumor therapy.

A large number of potent and differentially selective compounds have been designed, synthesised and patented as TACE inhibitors for the putative therapy of inflammatory disorders. A relatively large number of compounds can decrease the levels of TNF-α in cell and animal assays and display good efficacy, potency and bioavailability in cell and animal models of inflammatory disease. Several high efficacy compounds, such as BMS-561392 (in phase II for rheumatoid arthritis), have been taken to phase I and phase II clinical trials but no TACE inhibitor has yet made it to market. Another approach is a dual inhibitory effect of inhibition of TACE and selected MPP's, like that of Ro 32-7315.

The soluble plasma test composition of the present invention has been shown to reduce the release of TNF-α secretion by monocytes upon an LPS challenge as described in Examples 4 & 6. The suggestion is that the soluble plasma test composition also inhibits TACE.
A direct measurement of human TACE activity in human recombinant insect Sf21 cells revealed that the soluble plasma test composition of the present invention inhibited the TACE activity with an IC₅₀ of 1.3% of the soluble plasma test composition solution (see Figure 9).

### EXAMPLE 10 CASPASE INHIBITION

The interleukin-1b converting enzyme, ICE, now renamed caspase-1, is a cysteine endoprotease. The enzyme directly cleaves pro-IL-1 to mature cytokine IL-1b that is released into the extracellular environment. To date more than ten caspases are known. Much evidence has been accumulated to suggest that inhibition of caspase-1 can directly lead to a lowering of IL-1b in vitro and in vivo. This effect has been correlated with efficacy in ameliorating the symptoms of inflammation in many models of inflammatory diseases in animals and humans. Clinical trial data on pralnacasan and VX-765 have shown that caspase-1 inhibitors in general, can be effective for the treatment rheumatoid arthritis, osteoarthritis and psoriasis. Other pharmacological studies have also indicated that these inhibitors could be beneficial as therapeutic agents for a number of other disease states such as ischaemia/reperfusion injury and stroke. Of the few inhibitors that have entered clinical trials, all are reversible covalent (eg. aldehydes, pralnacasan and VX765) or irreversible inhibitors (eg. acyloxymethyl ketone 45). One possible problem with these compounds is their inherent reactive nature, which is not generally considered to be a desirable drug-like quality.

Caspases play a crucial role in mediating apoptosis. Thirteen members of the human caspase family have been identified. Some are involved in apoptosis, and these can be divided into two subgroups. The first group consists of caspase 8, caspase 9, and caspase 10, which function as initiators of the cell death process. The second group contains caspase 3, caspase 6, and caspase 7, which work as effectors, cleaving various substrates that ultimately cause the morphological and biochemical changes seen in apoptotic cells.

Apoptosis is a cellular response to a cellular insult such as UV light, chemical or physical damage or a viral infection. This insult initiates a cascade of events which lead to the destruction of the cell, often called "programmed cell death". It is an innate response of the cell which protects the rest of the organism. Exaggeration of apoptosis causes tissue-damage. Hepatitis, insulitis, graft-versus-host disease, and allergic encephalitis are due to the excessive apoptosis by the Fas ligand expressed on cytotoxic lymphocytes. Apoptotic cells are detected in the brain of ischemia or Alzheimer patients, suggesting that apoptosis is at least in part responsible for the disease manifestation. In stroke evidence has accumulated that neurons in the ischemic penumbra undergo apoptosis. In CD95 (APO-1/Fas)-deficient mice and in TNF-α-deficient mice, cerebral ischemic lesions were less. Mice injected with a mixture of neutralizing anti-TNF and anti-CD95L antibodies 30 min after induction of stroke have been reported to show a marked decrease in both infarct volumes and mortality. In the brain of Parkinson patients increased levels of inflammatory cytokines like caspase 1 and 3 have been reported.

Of all the known caspases, caspase-3 is.believed to be the primary executioner of apoptosis. Activation of caspase-3, depending on the activating mechanism, can induce chromatin condensation, DNA fragmentation, and cleavage of the DNA repair enzyme poly (ADP-ribose) polymerase, and eventuate in programmed cell death. An inhibition of caspase-3 can directly block cell apoptosis *in vitro.* In animal models of Alzheimer's disease and traumatic brain injury, pharmacological caspase-3 inhibition reduced the extent of brain damage as well as suppressed the number of Aβ deposits.

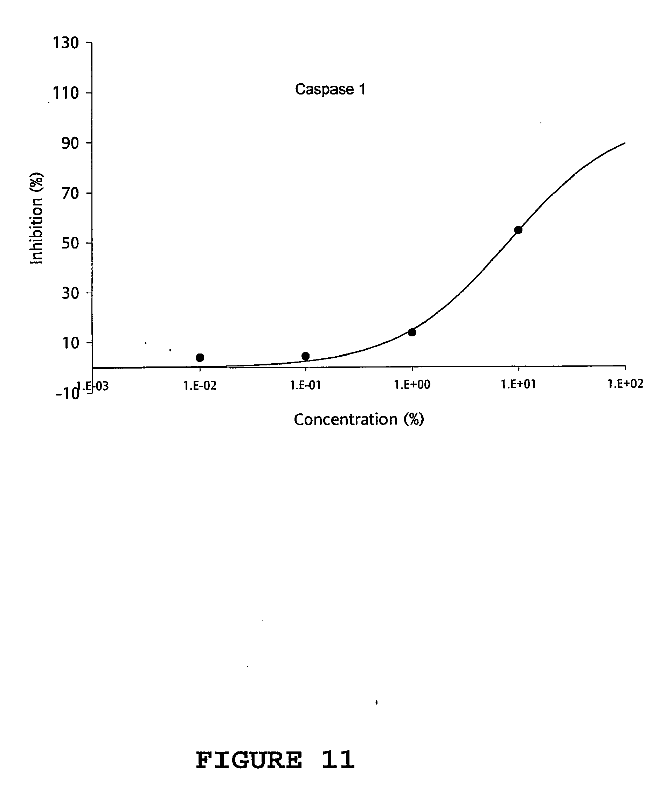

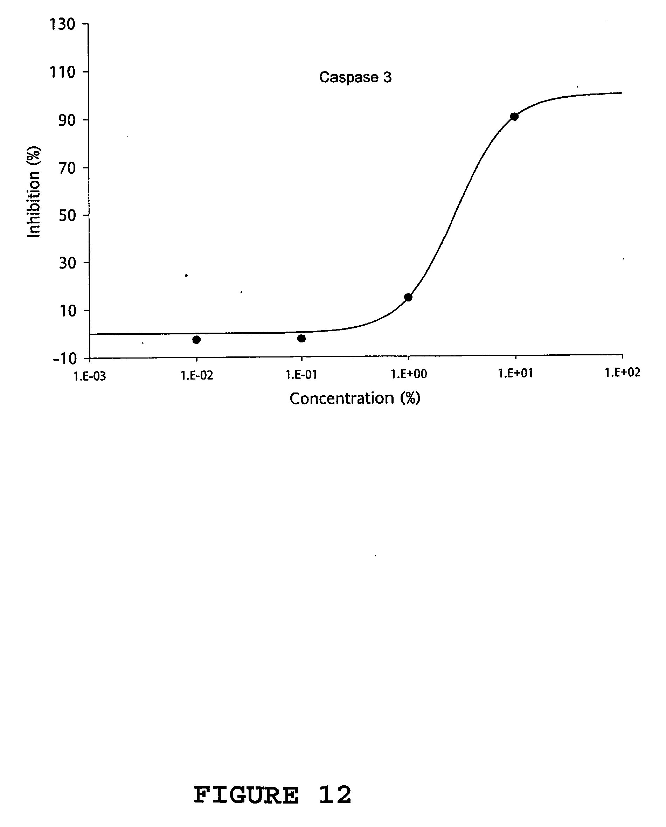

The soluble plasma test composition of the present invention was found to inhibit the human Caspase 1 with an IC₅₀ of 8.1% of the soluble plasma test composition solution. The Caspase 3 was inhibited with an IC₅₀ of 2.8% of the soluble plasma test composition solution. The Caspase 9 was inhibited 57% by 10% of the soluble plasma test composition solution (See Figures 11 & 12).

The inhibitory effects of the soluble plasma test composition on different caspases motivates an extensive investigation of the various caspases and the potential beneficial activity of soluble plasma test composition in apoptosis.

### SUMMARY

Table 5 shows the targets and the effects shown by the soluble plasma test composition of the present invention.

**TABLE 5**

| Target | Effect | Effect of 10% test composition | Kᵢ | IC₅₀ |
|---|---|---|---|---|
| TNF-α receptor | Binding | 50% | 7.2% | 10% |
| TACE inhibition | Inhibition | 100% | | 2.5% |
| TACE | Inhibition | 95% | | 1.3% |
| caspase-1 | Inhibition | 55% | | 8.1% |
| Caspase-3 | Inhibition | 90% | | 2.8% |
| Caspase-9 | Inhibition | 57% | | 2.8% |

## Claims

1. A composition for use in treatment of wounds in a subject by topical administration, wherein the composition comprises at least a fraction of a wound healing mixture, wherein said wound healing mixture is produced by:
- mixing plasma and/or serum with at least one metal, metal ion or metal salt thereto and heating the resulting mixture to at least 50°C;
- adding one or more proteases;
- heating the resultant mixture to a temperature of 80°C to 150°C; and
- allowing the mixture to cool to produce the wound healing mixture.

2. A composition according to claim 1, wherein the plasma or serum is isolated from an animal selected from the group consisting of human, equine, bovine, ovine, murine, caprine and canine.

3. A composition according to any one of claims 1 to 2, wherein the metal is selected from the group consisting of nickel, sodium, copper, zinc, cobalt, iron, magnesium, manganese, potassium, silver and mercury, ions or salts thereof and mixtures thereof.

4. A composition according to any one of claims 1 to 3, wherein the composition further comprises a pharmaceutical carrier.

5. A composition according to any one of claims 1 to 4, wherein the protease is selected from the group consisting of trypsin, chymotrypsin, factor Xa, venom-protease, thrombin, plasmin and a serine-protease of the subtilisin family.

6. A composition according to any one of claims 1 to 5, wherein the protease is trypsin.

7. A composition according to any one of claims 1 to 6, wherein the plasma and/or serum is dried and lyophilised before use.

8. A wound dressing comprising a composition of claim 1 to 7 for use in treatment of wounds.

## Patentansprüche

1. Zusammensetzung zur Anwendung bei der Behandlung von Wunden bei einem Patienten durch topische Verabreichung, wobei die Zusammensetzung mindestens einen Anteil einer Wundheilmischung umfasst, wobei die Wundheilmischung hergestellt wird durch:
- Mischung von Plasma und/oder Serum mit mindestens einem Metall, Metallion oder Metallsalz und Erwärmung der entstandenen Mischung auf mindestens 50°C,
- Zugabe einer oder mehrerer Proteasen,
- Erwärmung der entstandenen Mischung auf eine Temperatur von 80°C bis 150°C und
- Abkühlenlassen der Mischung zur Herstellung der Wundheilmischung.

2. Zusammensetzung nach Anspruch 1, wobei das Plasma oder Serum aus einem Tier isoliert wird, das aus der Gruppe bestehend aus Mensch, Pferd, Rind, Schaf, Maus, Ziege und Hund ausgewählt wird.

3. Zusammensetzung nach einem der Ansprüche 1 bis 2, wobei das Metall aus der Gruppe bestehend aus Nickel, Natrium, Kupfer, Zink, Kobalt, Eisen, Magnesium, Mangan, Kalium, Silber und Quecksilber, deren Ionen oder Salzen und Mischungen daraus ausgewählt wird.

4. Zusammensetzung nach einem der Ansprüche 1 bis 3, wobei die Zusammensetzung ferner einen pharmazeutischen Träger umfasst.

5. Zusammensetzung nach einem der Ansprüche 1 bis 4, wobei die Protease aus der Gruppe bestehend aus Trypsin, Chymotrypsin, Faktor Xa, Tiergift-Protease, Thrombin, Plasmin und einer Serinprotease der Subtilisinfamilie ausgewählt wird.

6. Zusammensetzung nach einem der Ansprüche 1 bis 5, wobei die Protease Trypsin ist.

7. Zusammensetzung nach einem der Ansprüche 1 bis 6, wobei das Plasma und/oder Serum vor der Verwendung getrocknet und lyophilisiert wird.

8. Wundverband, umfassend eine Zusammensetzung von Anspruch 1 bis 7 zur Anwendung bei der Behandlung von Wunden.

## Revendications

1. Composition à employer dans le traitement de plaies chez un sujet par application topique, la composition comprenant au moins une fraction d'un mélange de cicatrisation, ledit mélange de cicatrisation étant produit par :
- l'action de mélanger du plasma et/ou du sérum avec au moins un métal, un ion métallique ou un sel métallique et de chauffer le mélange résultant à au moins 50 °C ;
- l'addition d'une ou de plusieurs protéases ;
- le chauffage du mélange résultant à une température de 80 °C à 150 °C ; et
- le fait de laisser le mélange refroidir pour produire le mélange de cicatrisation.

2. Composition selon la revendication 1, le plasma ou le sérum étant isolé à partir d'un animal sélectionné dans le groupe constitué d'humains, d'équins, de bovins, d'ovins, de murins, de caprins et de canins.

3. Composition selon l'une quelconque des revendications 1 à 2, le métal étant sélectionné parmi le groupe constitué de nickel, sodium, cuivre, zinc, cobalt, fer, magnésium, manganèse, potassium, argent et mercure, d'ions ou de sels de ceux-ci et de mélanges de ceux-ci.

4. Composition selon l'une quelconque des revendications 1 à 3, la composition comprenant également un support pharmaceutique.

5. Composition selon l'une quelconque des revendications 1 à 4, la protéase étant sélectionnée parmi le groupe constitué de trypsine, chymotrypsine, facteur Xa, protéase de venin, thrombine, plasmine et d'une sérine protéase de la famille des subtilisines.

6. Composition selon l'une quelconque des revendications 1 à 5, la protéase étant une trypsine.

7. Composition selon l'une quelconque des revendications 1 à 6, le plasma et/ou le sérum étant séché et lyophilisé avant utilisation.

8. Pansement de plaie comprenant une composition des revendications 1 à 7 destinée au traitement de plaies.
